# EUROPEAN PATENT SPECIFICATION

(11) **EP 2 736 519 B1**
(45) Date of publication and mention of the grant of the patent: **13.04.2022**
(21) Application number: 12818358.9
(22) Date of filing: 27.07.2012
(51) Int. Cl.: A61K 45/06, A61Q 17/00, A61K 8/11, A61K 8/19, A61K 8/02, A61Q 11/00, A61K 8/34, A61K 6/52, A61K 8/86, A61K 6/54, A61K 6/69, A61P 31/04

(54) **ALKALINE COMPOSITIONS AND THEIR DENTAL AND MEDICAL USE**
ALKALISCHE ZUSAMMENSETZUNGEN UND IHRE ZAHNÄRZTLICHE UND SONSTIGE MEDIZINISCHE VERWENDUNG
COMPOSITIONS ALCALINES ET LEUR UTILISATION DENTAIRE ET MÉDICALE

(30) Priority: 27.07.2011 AU 2011902984
(43) Date of publication of application: 04.06.2014
(73) Proprietor: B Athanassiadis Dental Pty Ltd, Annerley, Queensland 4103 (AU); LJ Walsh Dental Pty Ltd, Ferny Hills, Queensland 4055 (AU)
(72) Inventor: ATHANASSIADIS, Basil, Annerley Brisbane, Queensland 4103 (AU); WALSH, Laurence James, Ferny Hills, Queensland 4055 (AU)
(74) Representative: Mewburn Ellis LLP
(86) International application number: PCT/AU2012/000895
(87) International publication number: WO 2013/013275

(56) References cited:
- EP-A1- 0 408 455
- EP-A2- 0 464 545
- WO-A1-94/11319
- WO-A1-2012/065946
- WO-A1-2012/075539
- WO-A2-02/15848
- WO-A2-2009/067774
- WO-A2-2012/031785
- US-A- 4 028 325
- US-A- 5 540 766
- US-A1- 2009 130 150
- US-A1- 2010 239 690
- YURI W. CAVALCANTI ET AL.: "Antimicrobial activity and pH evaluation of Calcium Hydroxide associated with natural products", BRAZ DENT SCI, vol. 13, no. 8, 2010, - 2010, pages 49-54, XP002734701,
- NOBUKE H ET AL: "The effect of calcium hydroxide eugenol agents on pulp of primary teeth of young dogs. The view of early stages", SHONI-SHIKAGAKU-ZASSHI : OFFICIAL PUBLICATION OF THE JAPANESE SOCIETY OF PEDIATRIC DENTISTRY = THE JAPANESE JOURNAL OF PEDIATRIC DENTISTRY, NIHON SHONI SHIKA GAKKAI, JP, vol. 27, no. 4, 1 January 1989 (1989-01-01), pages 915-921, XP008173532, ISSN: 0583-1199
- NOBUKE H. ET AL.: 'The effect of calcium hydroxide eugenol agents on pulp of primary teeth of young dogs. The view of early stages' SHONI SHIKAGAKU ZASSHI vol. 27, no. 4, 1989, pages 915 - 921, XP008173532
- ROCAS ISABELA N. ET AL.: 'In vivo antimicrobial effects of endodontic treatment procedures as assessed by molecular microbiologic techniques' JOURNAL OF ENDODONTICS vol. 37, no. 3, 2011, pages 304 - 310, XP028141361
- BARBOSA S., V. ET AL.: 'Cytotoxicity of endodontic irrigants containing calcium hydroxide and sodium lauryl sulphate on fibroblasts derived from mouse L929 cell line' BRAZILIAN DENTAL JOURNAL vol. 20, no. 2, 2009, pages 118 - 121, XP055143039

## Description

### Field of the Invention

The present invention relates generally to alkaline compositions that have antimicrobial activity. The present invention also relates to methods of making the compositions.

### Background of the Invention

The reference in this specification to any prior publication (or information derived from it), or to any matter which is known, is not, and should not be taken as an acknowledgment or admission or any form of suggestion that that prior publication (or information derived from it) or known matter forms part of the common general knowledge in the field of endeavour to which this specification relates.

Endodontic treatment in both humans and animals, seeks to avoid extracting damaged, infected or diseased teeth. Normally dental pulp is sterile, and if this tissue becomes infected or necrotic it can become a potential reservoir for bacteria. Since bacteria play a primary role in the initiation and progression of endodontic diseases, the presence of bacteria within the root canal system is linked fundamentally to poor clinical outcomes. Therefore, endodontic treatment aims to eradicate the bacteria inside teeth, seal the root canals and prevent recontamination of dental tissues.

One of the first steps in endodontic treatment is disinfection of the root canal by mechanical debridement of the canal wall and subsequent application of an antiseptic substance within the canal to kill the remaining bacteria. However, it is not possible to create a sterile space in all teeth with infected root canals due to the complexity of root canal systems, and the inability of instruments to contact all surfaces of the root canals (Byström and Sundqvist, Scandinavian Journal of Dental Research 1981;89:321-8; Byström et al Endodontic and Dental Traumatology 1985;1:70-5).

In particular, the root canal includes irregular spaces such as isthmuses, ramifications, accessory canals and apical deltas. Furthermore, the wall surrounding a root canal consists of dentine, a calcified organic matrix through which approximately 30,000 tubules/square mm pass horizontally from the root canal space towards the outer surface of the root. These tubules typically have a diameter of 2-5 micrometres, are not uniform in shape or size throughout the length of the root, and are also filled with organic matter. Bacteria which have a diameter of 1 micron easily penetrate, survive and even proliferate in these tubular structures as well as in the root canal and irregular spaces.

This is further complicated because root canal infections are often polymicrobial. Typically these infections include one or more bacteria selected from *Peptostreptococcus* spp., *Prevotella* spp., *Porphyromonas* spp., *Fusobacterium* spp., *Eubacterium* spp., *Actinomyces* spp., *Bacteroides* spp. and facultative *Streptococcus* spp. In cases of infected canals from previously root filled teeth the most common bacteria found include *Enterococcus* spp., *Streptococcus* spp. and *Lactobacillus* spp. The bacteria may be found not only in planktonic forms, but also as adherent well-organised biofilms which are more resistant to antimicrobial agents.

After mechanical debridement and application of an antiseptic, the use of intracanal medicaments is therefore recommended to prevent repopulation of the root canals with residual bacteria. It is also recommended to leave the medicament in the root canal for substantial periods of time, which means that the treatment of infected root canals is completed in more than one visit (Byström et al Endodontics and Dental Traumatology 1985;1:70-5; Chong and Pitt Ford, International Endodontic Journal 1992;25:97-106).

Intracanal medicaments should therefore include an antimicrobial agent which is broad spectrum covering Gram-negative bacteria, Gram-positive bacteria and fungi. Furthermore, the medicament needs to be easily removed, as after a period of treatment the medicament is replaced with an obturant to permanently fill the root canal. None of the current commercial products meet these requirements.

Obturants are used to permanently fill the root canal after treatment with a medicament. Obturants must be resorbable if they are to be used with deciduous teeth. Furthermore obturants need to set hard enough to provide a stable situation in the root canal, but not be difficult to work with and to remove, if required. Many commercially available obturants do not meet these requirements. For example, mineral trioxide aggregate (MTA) is not resorbable and therefore cannot be used with deciduous teeth as an endodontic obturant within the root canals. Zinc oxide eugenol (ZOE) is one of the most commonly used obturants in deciduous teeth, but ZOE has limited antimicrobial action, has a slow rate of resorption, has a tendency to be retained even after tooth exfoliation, and in some cases unresorbed material has been found to cause deflection of the succedaneous permanent tooth. Iodoform pastes, such as Kripaste, may also be used as obturants in deciduous teeth. However, Kripaste can be difficult to remove as it does not set hard, and iodoform pastes may produce a yellowish-brown discoloration of tooth crowns, which may compromise aesthetics.

There is a need to provide medicament compositions which have broad spectrum antibacterial activity and are easily removed after use as well as obturant compositions that are hard setting but able to be worked with and removed, have antimicrobial properties, if necessary, are resorbable and do not compromise aesthetics.

Similar considerations with regard to the need for broad spectrum antibacterial activity arise with regard to dental materials used to line deep cavities where they may be microscopic or macroscopic exposures of the dental pulp tissues. Because the lactic acid-affected dentine underlying a deep cavity is a low pH environment where acid-tolerant bacteria flourish, an ideal lining material for deep cavities in teeth would have high release of hydroxyl ions and would create an alkaline pH which would be highly unfavourable for the continued viability of acid-tolerant bacteria.

Current materials used to line deep cavities or which are placed on exposed dental pulp tissue are based on calcium hydroxide in water-based vehicles, which is problematic because the low solubility of calcium hydroxide in water limits the release of hydroxyl ions. MTA and related materials such as Biodentine (Septodont, Paris) which are based on tricalcium silicate and calcium oxide have been introduced recently, as derivatives from the chemistry of industrial Portland cement. The major limitations of these materials are their complex and expensive manufacturing processes, their long setting times, their awkward handling properties during manipulation by the dentist, and stringent requirements in terms of the ratio of water to powder. Biodentine cannot be mixed by hand, while MTA has the additional problem of causing discolouration of tooth structure. Because of such issues, there is a need for materials which have alkaline properties, are suitable for use in deep cavities, are easy to manipulate by the dentist, and do not cause staining of teeth. Such endodontic compositions are known from e.g. EP 0464545 A2, EP 0408455 A1 and Y.W. Cavalcanti et al., Braz. Dent. Sci (2010), vol. 13, no. 8, pg. 49-54.

Bone cements are used to anchor artificial joints, such as hip, knee, shoulder and elbow joints, in place. A risk with any type of surgery, is infection at the surgical site with bacteria or fungi. Therefore there is a need for bone cements with antimicrobial properties that reduce the risk of infection.

The present invention is predicated in part on the discovery that calcium hydroxide, either present in the composition or generated *in situ,* can be combined with solvents such as glycerol, propylene glycol and polyethylene glycol to provide liquid or paste compositions that contain higher amounts of calcium hydroxide than can be contained in aqueous compositions and thereby maintain greater hydroxyl ion release and higher pH in preparation and in use. Medicament compositions comprising calcium hydroxide and solvents such as polyethylene glycol or polypropylene glycol have a pH of at least 13 and have broad spectrum antimicrobial activity and desirable handling and storage properties because dehydration problems are eliminated. Obturant compositions formed from a calcium hydroxide source and a solvent such as glycerol or propylene glycol in approximately 1:1 ratio sets hard and has suitable properties for use as an obturant that has antimicrobial properties.

Similarly, chemical reactions which generate calcium hydroxide can be used not only for manufacturing suitable preparations for use as components in the compositions of the invention, but can directly contribute to the formation of suitable materials for root canal obturants and liners of deep cavities. Calcium hydroxide nanoparticles generated by chemical reactions can also be used in aqueous vehicles with co-solvents, allowing greater hydroxyl ion release, with use for rinsing root canals or as a dental mouthrinse. Appropriate selection of flavouring agents makes such compositions palatable and suitable for use even in individuals with low tolerance for conventional mouthrinses.

The present invention is also predicated in part on the discovery that a source of calcium hydroxide can be included in dental resins and bone cements to impart antimicrobial properties.

### Summary of the Invention

The invention is defined by claim 1. Further aspects are defined in the dependent claims.

In one aspect, the present invention provides an endodontic composition comprising a polyalkylene glycol solvent and calcium hydroxide, wherein the polyalkylene glycol solvent has a molecular weight of from 200 to 600 or a mixture thereof and is present in an amount of from 30% to 60% of the composition, and the calcium hydroxide is in an amount of from 25% to 55% of the composition; characterised in that the composition further comprises a viscosity modifier, wherein the viscosity modifier is a polyalkylene glycol with a molecular weight of from 2300 to 6000 or a mixture thereof, and the pH of the composition is at least 13.5. This composition is suitable for use as an endodontic medicament. In the description below this composition is referred to as "the medicament composition".

The medicament composition comprises calcium hydroxide in suitable solvent which is optionally lacking in water (water-free). Calcium hydroxide is bactericidal, is antifungal, can penetrate biofilms, can stimulate the repair of dentine and can withstand the buffering effect of dentine. In contrast, many endodontic medicaments comprise antibiotic/steroid combinations. Medicaments comprising antibiotic/steroid combinations are typically bacteriostatic, are not antifungal, have a limited ability to penetrate biofilms, are not involved in dentine repair, and their spectrum of activity is limited by the buffering effect of dentine. Furthermore, calcium hydroxide has the ability to kill a broader range of bacteria than antibiotic/steroid combinations. Bacteria have been reported to develop resistance to antibiotic/steroid combinations, in contrast to calcium hydroxide where resistance has not been reported.

While in some embodiments the calcium hydroxide may be used in granular or powder form having particulate size in the micron range or aggregates of such particles, in other embodiments, the calcium hydroxide may be in nanoparticulate form, having an average particulate size between 20 nanometers and 500 nanometers. Advantageously, the use of nanoparticulate calcium hydroxide increases the surface area of the calcium hydroxide and therefore maintains a highly alkaline pH even under dilution conditions, and improves dissolution of the calcium hydroxide into the polyalkylene glycol.

The nanoparticulate calcium hydroxide can be prepared by methods known in the art. For example, small quantities may be prepared by heating solutions of calcium chloride and sodium hydroxide to between 40°C to 90°C and then slowly adding the sodium hydroxide solution, for example, dropwise, to the warm calcium chloride solution. Another method is described by Danielea and Taglieri, Journal of Cultural Heritage, 2011, doi:10.1016/j.culher.20111.05.007). This method allows the bulk addition of hot calcium chloride solution to hot sodium hydroxide solution by addition of a non-ionic or anionic surfactant to the calcium chloride solution. The non-ionic surfactant may be any surfactant from the Tween, Triton or Brij series, ethoxylates based on polyoxyethylene or glycosides such as thioglycosides or maltosides (HEGA and MEGA series surfactants). Suitable anionic surfactants include sodium dodecyl sulfate, ammonium lauryl sulfate and sodium dodecyl benzene sulfonate, especially sodium dodecyl sulfate.

The surfactant present is present in the calcium chloride solution in an amount of from 0.1 to 2.0% w/w of the calcium chloride solution, especially about 1.0% w/w.

The nanoparticulate calcium hydroxide may be isolated by decanting the supernatant from the particulate or by filtration. The calcium hydroxide nanoparticulate material may also be washed with water to remove excess calcium chloride, sodium hydroxide and surfactant.

The medicament composition comprises from 25% to 55% calcium hydroxide, more especially from 30% to 50% calcium hydroxide, most especially from 35% to 45% calcium hydroxide.

While calcium hydroxide has previously been used in endodontic medicaments, such medicaments typically have a pH of below 12.6. For example, calcium hydroxide pastes (20-50%) in water with barium sulfate and methyl cellulose or carboxymethylcellulose have a pH in the range of 11.8 to 12.6. The dentine in the root canal buffers this type of medicament lowering the pH after application to about 7-8. While not wishing to be bound by theory, it is thought that the presence of a polyalkylene glycol solvent in the medicament composition results in a higher pH from a greater concentration of available hydroxide ions, hence a higher pH will be achieved once the medicament composition is buffered by the dentine and this will maintain the antimicrobial properties of calcium hydroxide.

The pH of the medicament composition is at least 13.5, more especially at least 13.7, and most especially at least 14.0. Without wishing to be bound by theory, it is believed that calcium hydroxide is more soluble in polyalkylene glycols than in water and therefore a greater pH can be achieved. Furthermore, the 0-14 pH range is relevant to acidic and basic aqueous solutions at room temperature. pH values greater than 14 can be achieved in nonaqueous compositions for example, pH values of 14.11 have been observed with compositions of calcium hydroxide in polyethylene glycol.

Advantageously, calcium hydroxide may be formulated into a medicament composition using a polyalkylene glycol solvent. The medicament composition of the present invention comprising a polyalkylene glycol solvent typically does not dry out when stored, and may be easily handled prior to and during administration. Many known endodontic medicaments use methylcellulose as a base, which tends to dry out over time. Furthermore, endodontic medicaments comprising methylcellulose may not be stable for extended periods of time.

The polyalkylene glycol solvent in the medicament composition is any polyalkylene glycol solvent which is in liquid form. The polyalkylene glycols are those recognised as safe for use in medical or food applications. In particular embodiments, the polyalkylene glycol is selected from polyC₂-C₃alkylene glycols, for example, polyethylene glycol (PEG) and polypropylene glycol (PPG). Polyalkylene glycols are polymeric ethers and therefore come in a variety of different molecular weights. Polyalkylene glycols may have a number that approximately corresponds to their molecular weight. The polyalkylene glycol solvent has a molecular weight of from 200 to 600 or a mixture thereof; more especially a polyalkylene glycol with a molecular weight of from 300 to 500 or a mixture thereof; most especially polyalkylene glycol 400. The polyalkylene glycol solvent may be selected from PEG 200, PEG 300, PEG 400, PEG 500, PEG 600, PPG 200, PPG 300, PPG 400, PPG 500 and PPG 600 or a combination thereof; more especially from PEG 300, PEG 400, PEG 500, PPG 300, PPG 400 and PPG 500 or a combination thereof; most especially PEG 400 or PPG 400.

The medicament composition comprises from 30 to 60% of the polyalkylene glycol solvent; especially from 35% to 55%; more especially from 40% to 50%; or from 42-48%; most especially about 45% of polyalkylene glycol solvent.

The medicament composition further comprises a viscosity modifier. The viscosity modifier is a polyalkylene glycol polymer with a molecular weight of from 2300 to 6000 or a mixture thereof. In some embodiments, the viscosity modifier is a PEG or PPG with a molecular weight of from 2300 to 6000 or a mixture thereof; especially a PEG or PPG with a molecular weight of from 2600 to 4000 or a mixture thereof; or a PEG or PPG with a molecular weight of from 2800 to 4000 or a mixture thereof; or a PEG or PPG with a molecular weight of from 3000 to 3750 or a mixture thereof; more especially a PEG or PPG with a molecular weight of from 3250 to 3500 or a mixture thereof; most especially PEG 3350 or PPG 3350. Exemplary polyalkylene glycol viscosity modifiers include PEG 2300, PEG 2400, PEG 2500, PEG 2600, PEG 2700, PEG 2800, PEG 2900, PEG 3000, PEG 3250, PEG 3350, PEG 3500, PEG 3750, PEG 4000, PPG 3000 and PPG 4000.

Use of higher molecular weight viscosity modifiers, especially PEG, provides medicament compositions with higher viscosities and these compositions may become firm if maintained for several hours at 2-4 °C. Such medicament compositions may be suitable for use in pastilles.

While not wishing to be bound by theory, it is believed that the viscosity of the medicament composition affects the rate of ionic dissociation of calcium hydroxide. Typically the lower the viscosity of the composition, the higher the rate of ionic dissociation of calcium hydroxide and the greater the hydroxyl ion release. Higher viscosity compositions may minimise the physical dispersion of calcium hydroxide medicament into the dental tissues and maintain the paste in the desired area for longer periods of time.

In some embodiments, the medicament composition comprises from 1% to 30% of a viscosity modifier. If a low viscosity product is desired, the medicament composition may comprise from 1% to 10 %; especially from 2% to 7.5%; more especially from 3% to 5%; most especially about 4% viscosity modifier.

For a medium viscosity composition, the medicament composition may comprise from 5 to 20% viscosity modifier; more especially from 7 to 18% viscosity modifier; or from 10 to 15% viscosity modifier; most especially about 12.5% viscosity modifier.

If a high viscosity composition is desired, the medicament composition may comprise from 15% to 30% viscosity modifier; especially from 17% to 28%; more especially from 20% to 25%; most especially about 22.5% viscosity modifier.

The viscosity may also be modified by the molecular weight of the viscosity modifier. For example, a high molecular weight viscosity modifier such as PEG 6000 will increase viscosity to a greater extent than a lower molecular weight viscosity modifier such as PEG 2300. The desired viscosity may be obtained by selecting a specific amount of a specific molecular weight viscosity modifier. Variation in viscosity may be achieved by varying the amount and/or molecular weight of the viscosity modifier.

The medicament composition may also further comprise a radiopaquing agent. Exemplary radiopaquing agents include strontium, zirconium, lanthanum, tungsten, bismuth or barium compounds; especially zirconium or barium compounds; more especially zirconium compounds. In some embodiments, the radiopaquing agent is selected from strontium oxide, zirconium silicate, zirconium oxide, zirconium dioxide, lanthanum oxide, calcium tungstate, bismuth oxide, barium zirconate and barium sulphate or a combination thereof; more especially zirconium dioxide and barium sulphate; most especially zirconium dioxide.

In some embodiments, the medicament composition comprises from 10% to 20% radiopaquing agent, especially from 12% to 18% radiopaquing agent; more especially about 15% radiopaquing agent.

The medicament composition may also further comprise an essential oil. Suitable essential oils include terpenes and terpenoids, for example, terpen-4-ol, cymene, sabinene, alpha pinene, beta pinene, citronellol, geraniol, carvacrol, thymol, farnesol and caryophyllene, and aromatic and aliphatic essential oils, for example, cinnamaldehyde, cinnamyl alcohol, chavicol, eugenol, amethole, estragole, safrole, ascaridole and menthol. In some embodiments, the composition comprises a mixture of essential oils. In particular embodiments, the essential oil has both antimicrobial and/or anti-inflammatory properties. In particular embodiments, the essential oil comprises terpinen-4-ol, cinnamaldehyde or carvacrol, or a mixture thereof. In one embodiment, the medicament composition may comprise up to 25% essential oil, especially up to 20% essential oil, or up to 15% essential oil, or up to 10% essential oil. For example, the medicament composition may comprise from 2% to 25% essential oil, or from 5% to 20% essential oil, or from 10% to 15% essential oil. In some embodiments, the medicament composition comprises from 2% to 25%, especially 10% to 15% terpinen-4-ol, or up to 10% carvacrol or cinnamaldehyde. In some embodiments, the essential oil is solubilised in the composition by the addition of a surfactant, especially a non-ionic surfactant that can solubilise oils in hydrophilic substances. One particularly useful surfactant is Mysol^{™}381.

In one embodiment, the medicament composition further comprises an anti-inflammatory agent. In some embodiments, the anti-inflammatory agent is a nonsteroidal anti-inflammatory drug (NSAID) or a corticosteroid, especially a NSAID. Exemplary NSAIDs include ibuprofen, dexibuprofen and lysine ibuprofen, especially ibuprofen and dexibuprofen, more especially dexibuprofen.

The medicament composition may also further comprise a colourant. The colourant may produce a medicament with a white colour, and may be especially a titanium compound, for example, titanium dioxide. In some embodiments, the medicament composition comprises between 0.5% and 5% colourant, especially about 2% colourant.

In some embodiments, the medicament compositions may have a neutral, non-penetrating colour, especially if the composition comprises, for example, a colourant that is titanium dioxide, and a radiopaquing agent that is zirconium dioxide or barium sulphate.

In some embodiments, the medicament composition further comprises one or more vehicles. Exemplary vehicles are selected from glycerol; propylene glycol, acids such as oleic acid; water; ethanol; silicone-based materials (both non-volatile and volatile) such as cyclomethicone, dimethicone and dimethiconecopolyol; hydrocarbons such as squalane, squalene and petrolatum, especially squalane and petrolatum; a sustained release vehicle such as a microsponge or a polymer matrix; surfactants, such as cationic and amphoteric surfactants; a stabilising agent; a suspending agent; an emulsifying agent; a pH regulator such as a citrate or a phosphate salt; or a combination thereof. Other vehicles suitable for use with the compositions would also be known from the cosmetic and pharmaceutical arts.

Mixtures of water and organic solvents (such as water and ethanol), and mixtures of ethanol and glycerol or propylene glycol may also be present in the medicament composition. In some embodiments, the medicament composition comprises up to 5% water, especially up to 3% water, more especially up to 1% water, and most especially the medicament composition does not comprise water.

In another aspect, the present invention relates to a method of making the medicament composition described above. This method comprises adding calcium hydroxide to a polyalkylene glycol solvent and a viscosity modifier, and mixing the combination. This combination should be mixed for a period of at least 5 minutes to provide a homogenous composition. In other embodiments, this method further comprises mixing one or more of a radiopaquing agent, an essential oil, an anti-inflammatory agent, a colourant and a vehicle into the polyalkylene glycol solvent or into the calcium hydroxide-polyalkylene glycol solvent combination.

In one example, to provide a medium viscosity paste deionized water is optionally added to PEG 400 in a stainless steel vessel. Water typically comprises 0 - 5% of the total volume. PEG 3350 is then added, and the mixture is heated (for example to 50-70 °C) with constant stirring until the PEG 3350 melts and dissolves evenly into the mixture. The mixture is then removed from heating and the following are added (with gentle stirring), especially in sequence: calcium hydroxide powder, zirconium dioxide powder, and titanium dioxide powder. The mixture is then agitated mechanically for 5 minutes, and is then dispensed into tubes or syringes as appropriate.

In another aspect, the present invention provides the medicament composition described above for use in the treatment or prevention of a microbial infection in a tooth, painful extirpation, "hot pulp" syndrome or acute pulpitis, or a combination thereof; especially in the treatment or prevention of a microbial infection in a tooth.

Also disclosed herein, not forming part of the invention, is a method of treating or preventing a microbial infection in a tooth, painful extirpation, "hot pulp" syndrome or acute pulpitis, or a combination thereof (especially a microbial infection in a tooth), comprising administering the medicament composition described above to a subject. The medicament composition is especially administered into the root canal of the subject.

In particular embodiments, the microbial infection is caused by *Enterococcus* spp., *Streptococcus* spp., *Lactobacillus* spp., *Peptostreptococcus* spp., *Prevotella* spp., *Porphyromonas* spp., *Fusobacterium* spp., *Eubacterium* spp., *Actinomyces* spp., *Bacteroides* spp., and *Candida* spp., especially *Enterococcus* spp. such as *E. Faecalis* and *Candida* spp. such as *Candida albicans.*

As used throughout the specification, the term subject refers to a human or animal in which dental procedures are carried out or those in which bone defects would be repaired or joints replaced. The animal may be a high value animal such as a companion animal or pet or a horse such as a race horse or a captive wild animal such as an animal kept in a zoo. In particular embodiments, the subject is a human.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

Unless stated otherwise, the percentages appearing within this specification and the claims which follow represent percentages by weight.

The invention will now be described with reference to the following Examples which illustrate some aspects of the present invention. However, it is to be understood that the particularity of the following Examples is not to supersede the generality of the preceding description of the invention.

### Examples of the invention and Reference Examples not forming part of the invention

### Example 1: Endodontic Medicament Composition

PEG 400 liquid (76 grams) and PEG 3350 (4 grams) were added to a stainless steel vessel, and the resultant mixture was heated at 60 °C with constant stirring until the PEG 3350 had melted and dissolved evenly into the mixture, over a period of approximately 2 to 3 minutes.

The mixture was then removed from heat, and then the following powders were added in sequence with gentle stirring: (i) calcium hydroxide powder (65 grams), (ii) zirconium dioxide powder (25 grams), and (iii) titanium dioxide powder (3.5 grams). The final pH of the set product was measured 5 minutes after inserting a pH electrode, achieving a pH of 13.5.

The cooling mixture was then mechanically stirred for a period of 5 minutes, and then the mixture was dispensed into tubes or syringes.

### Example 2: Endodontic Medicament comprising terpinen-4-ol anti-inflammatory agent

The same procedure as Example 1 was followed with the exception that when the mixture was removed from the heat, the following components were added with gentle stirring: i) calcium hydroxide powder (72 g), ii) zirconium dioxide powder (33 g), iii) titanium dioxide powder (2.5 g) and iv) terpinen-4-ol (22 g).

### Example 3: Endodontic Medicament comprising dexibuprofen anti-inflammatory agent

The same procedure as Example 2 was followed with the exception that the terpinen-4-ol was replaced with dexibuprofen (22 g).

### Reference Example 4: Preparation of Calcium Hydroxide Nanoparticles

Calcium hydroxide nanoparticles were prepared based on the method of Danielea and Taglieri (Journal of Cultural Heritage (June 2011), doi:10.1016/j.culher.2011.05.007). Briefly, 4.41 g (0.03 moles of calcium chloride dihydrate was dissolved into 100 mL of deionized water, together with 1% w/w Tween 80 based on the weight of calcium chloride solution. A solution of sodium hydroxide was prepared by dissolving 2.4 g into 100 mL of water. The calcium chloride solution and sodium hydroxide solution were warmed separately to a constant temperature of 50°C then mixed together in bulk to give a milky solution of about 200 mL. The calcium hydroxide precipitates and the particles settle at the bottom of the container.

After 60 minutes, the supernatant was removed by decantation and the calcium hydroxide solid nanoparticles having an average size range of between 50 and 500 nm were washed with distilled water (100 mL) by agitation for 30 to 120 minutes. The wash water was then decanted. The pH of the nanoparticles of calcium hydroxide in a solution of 500 mL distilled water was greater than 12.

### Example 5: Endodontic Medicament Composition

The steps of Example 1 were repeated but the calcium hydroxide powder was replaced with 65 g of thick suspension of calcium hydroxide nanoparticles prepared in Reference Example 4. The final pH was 13.5.

Those skilled in the art will appreciate that the invention described herein is susceptible to variations and modifications other than those specifically described and the invention is defined by the claims.

## Claims

1. An endodontic composition comprising a polyalkylene glycol solvent and calcium hydroxide, wherein the polyalkylene glycol solvent has a molecular weight of from 200 to 600 or a mixture thereof and is present in an amount of from 30% to 60% of the composition, and the calcium hydroxide is in an amount of from 25% to 55% of the composition; **characterised in that** the composition further comprises a viscosity modifier, wherein the viscosity modifier is a polyalkylene glycol with a molecular weight of from 2300 to 6000 or a mixture thereof, and the pH of the composition is at least 13.5.

2. The composition according to claim 1, wherein the pH of the composition is at least 14.0.

3. The composition according to claim 1 or claim 2 wherein the polyalkylene glycol solvent is polyethylene glycol or polypropylene glycol.

4. The composition according to claim 1 or claim 2, wherein the polyalkylene glycol solvent is polyethylene glycol 400 or polypropylene glycol 400.

5. The composition according to any one of claims 1 to 4, wherein the viscosity modifier is a polyalkylene glycol with a molecular weight of from 2800 to 4000 or a mixture thereof.

6. The composition according to claim 5, wherein the viscosity modifier is polyethylene glycol 3350 or polypropylene glycol 3350.

7. The composition according to any one of claims 1 to 6 further comprising a radiopaquing agent.

8. The composition according to claim 7 wherein the radiopaquing agent is zirconium dioxide, barium sulphate or barium zirconate.

9. The composition according to any one of claims 1 to 8 further comprising at least one anti-inflammatory agent.

10. The composition according to claim 9 wherein the anti-inflammatory agent is selected from essential oils and NSAIDS, wherein the NSAID is selected from ibuprofen, dexibuprofen and lysine ibuprofen.

11. A method of making the composition defined in any one of claims 1 to 10 comprising adding calcium hydroxide to a polyalkylene glycol solvent and a viscosity modifier, and mixing the combination.

12. A composition according to any one of claims 1 to 10 for use in treating or preventing a microbial infection in a tooth, painful extirpation, "hot pulp" syndrome or acute pulpitis, or a combination thereof in a subject.

## Patentansprüche

1. Endodontische Zusammensetzung, die ein Polyalkylenglykol-Lösungsmittel und Calciumhydroxid umfasst, wobei das Polyalkylenglykol-Lösungsmittel ein Molekulargewicht von 200 bis 600 aufweist oder ein Gemisch mehrerer davon ist und in einer Menge von 30 % bis 60 % der Zusammensetzung vorliegt und das Calciumhydroxid in einer Menge von 25 % bis 55 % der Zusammensetzung vorliegt; **dadurch gekennzeichnet, dass** die Zusammensetzung außerdem einen Viskositätsmodifikator umfasst, wobei der Viskositätsmodifikator ein Polyalkylenglykol mit einem Molekulargewicht von 2300 bis 6000 oder ein Gemisch mehrerer davon umfasst und der pH der Zusammensetzung zumindest 13,5 beträgt.

2. Zusammensetzung nach Anspruch 1, wobei der pH der Zusammensetzung zumindest 14,0 beträgt.

3. Zusammensetzung nach Anspruch 1 oder Anspruch 2, wobei das Polyalkylenglykol-Lösungsmittel Polyethylenglykol oder Polypropylenglykol ist.

4. Zusammensetzung nach Anspruch 1 oder Anspruch 2, wobei das Polyalkylenglykol-Lösungsmittel Polyethylenglykol 400 oder Polypropylenglykol 400 ist.

5. Zusammensetzung nach einem der Ansprüche 1 bis 4, wobei der Viskositätsmodifikator ein Polyalkylenglykol mit einem Molekulargewicht von 2800 bis 4000 oder ein Gemisch mehrerer davon ist.

6. Zusammensetzung nach Anspruch 5, wobei der Viskositätsmodifikator Polyethylenglykol 3350 oder Polypropylenglykol 3350 ist.

7. Zusammensetzung nach einem der Ansprüche 1 bis 6, die außerdem ein Kontrastmittel umfasst.

8. Zusammensetzung nach Anspruch 7, wobei das Kontrastmittel Zirkoniumdioxid, Bariumsulfat oder Bariumzirkonat ist.

9. Zusammensetzung nach einem der Ansprüche 1 bis 8, die außerdem zumindest einen Entzündungshemmer umfasst.

10. Zusammensetzung nach Anspruch 9, wobei der Entzündungshemmer aus ätherischen Ölen und NSAIDs ausgewählt ist, wobei das NSAID aus Ibuprofen, Dexibuprofen und Lysin-Ibuprofen ausgewählt ist.

11. Verfahren zur Herstellung einer Zusammensetzung nach einem der Ansprüche 1 bis 10, welches das Zusetzen von Calciumhydroxid zu einem Polyalkylenglykol-Lösungsmittel und einem Viskositätsmodifikator sowie das Vermischen der Kombination umfasst.

12. Zusammensetzung nach einem der Ansprüche 1 bis 10 zur Verwendung bei der Behandlung oder Prävention einer mikrobiellen Infektion in einem Zahn, einer schmerzhaften Exstirpation, des Hot-Pulp-Syndroms, von akuter Pulpitis oder einer Kombination davon bei einem Individuum.

## Revendications

1. Composition endodontique comprenant un solvant de polyalkylène glycol et de l'hydroxyde de calcium, dans laquelle le solvant de polyalkylène glycol a un poids moléculaire de 200 à 600 ou un mélange de celui-ci et est présent en une quantité de 30 % à 60 % de la composition, et l'hydroxyde de calcium est en une quantité de 25 % à 55 % de la composition ; **caractérisée en ce que** la composition comprend en outre un modificateur de viscosité, dans laquelle le modificateur de viscosité est un polyalkylène glycol ayant un poids moléculaire de 2 300 à 6 000 ou un mélange de celui-ci, et le pH de la composition est d'au moins 13,5.

2. Composition selon la revendication 1, dans laquelle le pH de la composition est d'au moins 14,0.

3. Composition selon la revendication 1 ou la revendication 2, dans laquelle le solvant de polyalkylène glycol est le polyéthylène glycol ou le polypropylène glycol.

4. Composition selon la revendication 1 ou la revendication 2, dans laquelle le solvant de polyalkylèneglycol est le polyéthylèneglycol 400 ou le polypropylèneglycol 400.

5. Composition selon l'une quelconque des revendications 1 à 4, dans laquelle le modificateur de viscosité est un polyalkylèneglycol ayant un poids moléculaire de 2 800 à 4 000 ou un mélange de celui-ci.

6. Composition selon la revendication 5, dans laquelle le modificateur de viscosité est le polyéthylèneglycol 3 350 ou le polypropylèneglycol 3 350.

7. Composition selon l'une quelconque des revendications 1 à 6, comprenant en outre un agent rendant radio-opaque.

8. Composition selon la revendication 7, dans laquelle l'agent rendant radio-opaque est le dioxyde de zirconium, le sulfate de baryum ou le zirconate de baryum.

9. Composition selon l'une quelconque des revendications 1 à 8, comprenant en outre au moins un agent anti-inflammatoire.

10. Composition selon la revendication 9, dans laquelle l'agent anti-inflammatoire est choisi parmi les huiles essentielles et des AINS, dans laquelle l'AINS est choisi parmi l'ibuprofène, le dexibuprofène et l'ibuprofène lysine.

11. Procédé de préparation de la composition définie dans l'une quelconque des revendications 1 à 10, comprenant l'addition d'hydroxyde de calcium à un solvant de polyalkylèneglycol et à un modificateur de viscosité, et le mélange de la combinaison.

12. Composition selon l'une quelconque des revendications 1 à 10, à utiliser dans le traitement ou la prévention d'une infection microbienne dans une dent, d'une extirpation douloureuse, du syndrome de la « pulpe chaude » ou d'une pulpite aiguë, ou d'une combinaison de ceux-ci chez un sujet.
